# EUROPEAN PATENT SPECIFICATION

(11) **EP 1 545 449 B1**
(45) Date of publication and mention of the grant of the patent: **11.06.2008**
(21) Application number: 03767224.3
(22) Date of filing: 04.08.2003
(51) Int. Cl.: A61K 8/19, A61K 8/21, A61Q 11/00

(54) **DENTINAL DESENSITIZING DUAL COMPONENT DENTIFRICE**
DESENSIBILISIERENDE ZWEIKOMPONENTENZAHNPASTA
DENTIFRICE DESENSIBILISANT A DEUX COMPOSANTS

(30) Priority: 05.08.2002 US 212660; 07.01.2003 US 338160
(43) Date of publication of application: 29.06.2005
(73) Proprietor: Colgate-Palmolive Company, New York, N.Y. 10022 (US)
(72) Inventor: FISHER, Steven, W., Middlesex, NJ 08846 (US); JOZIAK, Marilou, T., South River, NJ 08882 (US); SULLIVAN, Richard, J., Somerset, NJ 08873 (US); STRANICK, Michael, A., Bridgewater, NJ 08807 (US); HEU, Rodman, T., Chatham, NJ 07928 (US)
(74) Representative: Winckels, Johannes Hubertus F.
(86) International application number: PCT/US2003/024526
(87) International publication number: WO 2004/012693

(56) References cited:
- WO-A-00/62749
- WO-A-01/45660
- WO-A-01/66074
- WO-A-99/53893
- WO-A-99/55297
- GB-A- 2 188 548
- US-A- 5 215 740
- US-A- 5 702 686
- US-A- 5 718 885
- US-A- 5 780 015
- US-A- 5 895 641
- US-A- 5 939 052
- US-B1- 6 180 089

## Description

### 1. Field of the Invention

The present invention relates to a desensitizing dentifrice composition which eliminates or reduces the discomfort and pain associated with dentinal hypersensitivity and more particularly to a desensitizing dental composition containing a potassium salt desensitizing agents which exhibits unexpected enhanced anticavity and remineralization properties.

### 2. The Prior Art

Dentinal hypersensitivity is defined as acute, localized tooth pain in response to physical stimulation of the dentine surface as by thermal (hot or cold) osmotic, tactile combination of thermal, osmotic and tactile stimulation of the exposed dentin.

Exposure of the dentine, which is generally due to recession of the gums, or loss of enamel, frequently leads to hypersensitivity. The art has determined that dentine tubules open to the surface have a high correlation with dentine hypersensitivity, Abs, J. Clin. Periodontal. 14,280-4 (1987). Dentinal tubules lead from the pulp to the cementum. When the surface cementum of the tooth root is eroded, the dentinal tubules become exposed to the external environment. The exposed dentinal tubules provide a pathway for transmission of fluid flow to the pulpal nerves, the transmission induced by changes in temperature, pressure and ionic gradients.

It is known to the art that potassium salts are effective in the treatment of dentinal hypersensitivity. For example, U.S. 3,863,006 discloses that toothpastes containing potassium salts such as potassium nitrate desensitize the teeth after tooth brushing for several weeks. It is believed by those skilled in the art that an elevation in the extracellular potassium concentration in the vicinity of pulpal nerves underlying sensitive dentin is responsible for the therapeutic desensitizing effect of topically applied oral products which contain potassium nitrate. Due to passive diffusion of potassium ion into and out of the open dentine tubules, repeated application of the active ingredient is necessary to build up the necessary concentration in the vicinity of the pulpal nerves.

It is believed that the improved pain relief is obtained from the use of potassium salts in combination with gradual mineralization on the dentin surface which can either totally or partially occlude dentin tubules. Total occlusion will dramatically reduce fluid flow within the tubules which stimulates pain. Partial occlusion of the dentin tubules is believed to increase delivery of potassium ion inside the tooth because the inward diffusive flux is less dependent upon tubule radius than outward fluid flow (due to positive pulpal pressures) (See DH Pashley and WG Mathews, Archs. Oral Biol. (1993) 38, 577-582). Therefore, this enhanced delivery of potassium should enhance relief.

It has also long been known to include fluoride releasing compounds in dentifrices as anticaries agents, and it has been established that these compounds are effective to reduce the incidence of dental caries. Fluoride compounds which are conventionally used are sodium fluoride, sodium monofluorophosphate and stannous fluoride. The fluoride compounds are effective mainly due to the fluoride ions which improve the acid resistance of tooth enamel and accelerate recalcification or remineralization of decayed teeth in their early stage when the demineralization has proceeded only slightly. By remineralization, pre-existing tooth decay and caries can be reduced or eliminated thereby reducing preexisting carious conditions in the tooth structure. The effect of improving the acid resistance of the enamel is believed to be due to the fact that the fluoride ions are incorporated into a crystal lattice of hydroxyapatite which is the main constituent of tooth enamel or, in other words, fluoride ions partially fluoridate hydroxyapatite and simultaneously repair the lattice irregularities.

The effectiveness of fluoride treatment is dependent upon the amount of fluoride ion which is available for deposition on the enamel being treated. It is, therefore, desirable to formulate dentifrice compositions which provide maximum fluoride ion availability in brushing solutions formed using the dentifrice.

While the prior art discloses the use of various oral compositions for the treatment of dentinal hypersensitivity, dental caries, and enamel demineralization there is still a need for additional compositions and methods which provide improved performance in such treatments.

### SUMMARY OF THE INVENTION

In accordance with the present invention there is provided a two component dental composition which eliminates or substantially reduces the discomfort and pain associated with dentinal hypersensitivity which composition comprises a first dentifrice component having a neutral pH in the range of 6.5 to 7.5, the pH being buffered with a phosphate salt, a second dentifrice component having an alkaline pH in the range of 8.0 to 9.9, and at least one of the components containing a fluoride and potassium ion releasable salt, the fluoride salt present in the composition delivering a fluoride ion concentration of 1100 to 8800 ppm, the first and second components being maintained separate from each other until dispensed and combined for application to teeth requiring relief from dentine hypersensitivity, whereby heightened desensitization is experienced by the user.

Further the invention is directed to the use of (1) a first dentifrice component having a neutral pH buffered with a phosphate salt in the range of 6.5 to 7.5 and (2) a second dentifrice component having an alkaline pH in the range of 8.0 to 9.9, at least one of the components containing a desensitizing potassium ion releasable salt and a fluoride ion releasable salt, for eliminating or reducing the discomfort and pain associated with dentinal hypersensitivity, the fluoride salt present in the composition delivering a fluoride ion concentration of 1100 to 8800 ppm, which first and second dentifrice components are in the manufacture of a composition separately housed , dispensing the first and second components and combining the dispensed components for application to teeth requiring relief from dentine hypersensitivity and thereafter applying the combined components to the teeth whereby heightened desensitization is experienced by the user.

### In the Drawings

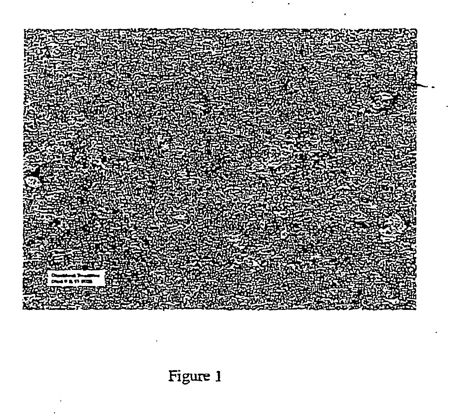

Fig. 1 is a SEM recorded at 2,000 x magnification, of a dentin disk surface treated with a dual component dentifrice containing high concentrations of a fluoride salt which releases 5000 ppm fluoride ion (1.1% by weight) and potassium nitrate (5% by weight), wherein the first component is buffered to a pH of 6.5 and the second component adjusted to a pH of 9.5 with sodium hydroxide, the pH of the combined components being 7.5 .

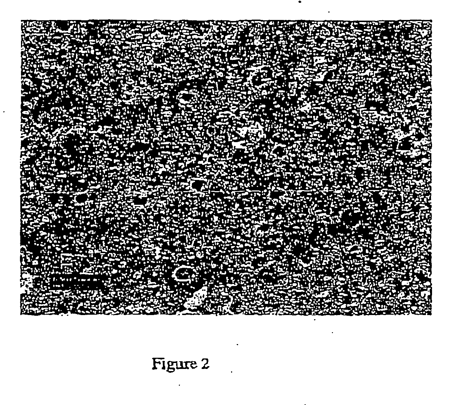

Fig.2 is a SEM recorded at 2,000 x magnification, of a dentin disk surface treated with the combined components of a comparative dual component dentifrice of the prior art (US 6,180,089) containing 5% potassium nitrate and a fluoride containing salt which releases 1100 ppm fluoride ion wherein one component is maintained at alkaline pH and the second component maintained at an acid pH the pH of the combined components being 7.0.

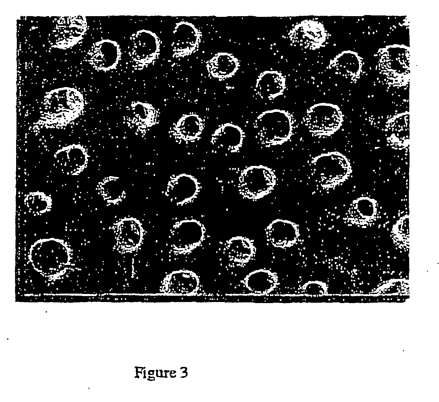

Fig. 3 is a scanning electron photomicrograph (SEM) recorded at 2,000 x magnification of a dentin disk surface treated with a phosphate buffer solution.

### DESCRIPTION OF THE PREFERRED EMBODIMENTS

The composition of the present invention is a dual phase composition.

The dual phase composition is comprised of two components in which a first dentifrice component is maintained at an alkaline pH of preferably 8.5 to 9.9 and more preferably 9.0 to 9.9, and a second dentifrice component is buffered to maintain the pH at a substantially neutral pH level of 6.5 to 7.0. The two components are preferably combined in approximately equal weight proportions, so that about one-half of the concentration of any particular ingredient within either component will be present when the components are combined and applied to the teeth, as by brushing. Both components are preferably formulated to have similar physical characteristics, so that the two components may be simultaneously delivered in the desired predetermined amounts by extrusion when separately housed in a multicompartmented tube or pump device.

In the dual component dentifrice of the present invention, the one dentifrice component is prepared having an alkaline pH and a composition otherwise similar to that of the other having a buffered neutral pH. The pH of the alkaline component is adjusted to a pH of preferably 8.5 to 9.7 and more preferably 9.0 to 9.5. The pH of the combined dentifrice components is in the range of 7.5 to 8.6 and preferably 7.5 to 8.5

An alkaline agent such as an alkali metal compound including sodium hydroxide, potassium hydroxide, sodium bicarbonate, sodium carbonate, N-sodium silicate a sodium silicate in 34.6% water (available from PQ Corporation) is incorporated in the alkaline pH dentifrice component of the dual component dentifrice in amounts in the range of 0.5 to 15% by weight, preferably 1.0 to 8% by weight and most preferably at 1.0 to 5.0% by weight of the component. Mixtures of the above alkali metal compounds may also be used. Sodium hydroxide is the preferred alkaline agent.

The humectant used in the preparation of the vehicle for the dentifrice composition of the present invention is generally a mixture of humectants, such as glycerol, sorbitol and a polyethylene glycol of molecular weight in the range of 200 to1000, but other mixtures of humectants and single humectants may also be employed. The humectant content is in the range of 10% to 50% by weight and preferably 20 to 40% by weight of the dentifrice component. The water content is in the range of 20 to 50% by weight and preferably 30 to 40% by weight.

Thickeners used in the preparation of the dentifrice vehicle include organic and inorganic thickeners. Inorganic thickeners which may be included in the dentifrice components include amorphous silicas such as Zeodent 165 available from Huber Corporation, and Sylox 15 from W.R. Grace.

Organic thickeners of natural and synthetic gums and colloids may also be used to prepare the dentifrice components of the present invention. Examples of such thickeners are carrageenan (Irish moss), xanthan gum, sodium carboxymethyl cellulose, starch, polyvinylpyrrolidone, hydroxyethylpropylcellulose, hydroxybutyl methyl cellulose, hydroxypropyl methyl cellulose, and hydroxyethyl cellulose.

The inorganic thickener may be incorporated in the dentifrice composition of the present invention at a concentration of 0.5 to 5% by weight and preferably 1 to 3% by weight. The organic thickener may be incorporated in the compositions of the present invention at a concentration of 0.1 to 3% by weight and preferably 0.4 to 1.5% by weight.

Surfactants may be incorporated in the dentifrice compositions to provide foaming properties. The surfactant is preferably anionic or nonionic in nature. Suitable examples of anionic surfactants are higher alkyl sulfates such as potassium or sodium lauryl sulfate which is preferred, higher fatty acid monoglyceride monosulfates, such as the salt of the monosulfated monoglyceride of hydrogenated coconut oil fatty acids, alkyl aryl sulfonates such as sodium dodecyl benzene sulfonate, higher fatty sulfoacetates, higher fatty acid esters of 1,2 dihydroxy propane sulfonate.

The surfactant agent is generally present in the dentifrice component composition of the present invention at a concentration of 0.5 to 10.0% by weight and preferably 1.0 to 5.0% by weight.

Abrasives may be incorporated in the dentifrice composition of the present invention and preferred abrasives are siliceous materials, such as silica. A preferred silica is a precipitated amorphous hydrated silica, such as Sorbosil AC-35, marketed by Crosfield Chemicals, or Zeodent 115 from Huber Company but other abrasives may also be employed, including hydroxyapatite, sodium metaphosphate, potassium metaphosphate, tricalcium phosphate, calcium phosphate dihydrate, anhydrous dicalcium phosphate, calcium pyrophosphate, magnesium orthophosphate, trimagnesium phosphate, calcium carbonate, sodium bicarbonate, alumina trihydrate, aluminum silicate, calcined alumina and bentonite.

The concentration of abrasive in the dentifrice composition of the present invention will normally be in the range of 5 to 40% by weight and preferably 10 to 25% by weight.

The source of desensitizing potassium ion is generally a water soluble potassium salt including potassium nitrate, potassium citrate, potassium chloride, potassium bicarbonate and potassium oxalate with potassium nitrate being preferred. The potassium salt is generally incorporated in one or more of the dentifrice components at a concentration of 1 to 20% by weight and preferably 3 to 10% by weight.

Fluoride ion releasing salts are incorporated in the dentifrice composition of the present invention and are characterized by their ability to release fluoride ions in water. It is preferable to employ a water soluble fluoride salt providing 100 to 8800 ppm of fluoride ion, and preferably 2500 to 8800 ppm of fluoride ion. Suitable examples of fluoride ion releasing salts include water soluble inorganic metal salts, for example, sodium fluoride, potassium fluoride, sodium monofluorophosphate, stannous fluoride and sodium fluorosilicate. Sodium fluoride, sodium monoflurophosphate and stannous fluoride are preferred fluoride ion releasing salts.

Pyrophosphate salts having anticalculus efficacy useful in the practice of the present invention include water soluble salts such as dialkali or tetraalkali metal pyrophosphate salts such as Na₄P₂O₇ (TSPP), K₄P₂O₇, Na₂K₂P₂O₇, Na₂H₂P₂O₇ and K₂H₂P₂O₇. Polyphosphate salts include the water soluble alkali metal tripolyphosphates such as sodium tripolyphosphate and potassium tripolyphosphate.

The pyrophosphate salts are incorporated in the dentifrice composition of the present invention at a concentration of 0.5 to 2.0% by weight, and preferably 1.5 to 2% by weight and the polyphosphate salts are incorporated in the dentifrice composition of the present invention at a concentration of 1.0 to 7.0% by weight.

Colorants such as pigments and dyes may be used in the practice of the present invention. Pigments include nontoxic, water insoluble inorganic pigments such as titanium dioxide and chromium oxide greens, ultramarine blues and pinks and ferric oxides as well as water insoluble dye lakes prepared by extending calcium or aluminum salts of FD&C dyes on alumina such as FD&C Green #1 lake, FD&C Blue #2 lake, FD&C R&D #30 lake and FD&C #Yellow 15 lake. The pigments have a particle size in the range of 5-1000 µm (microns), preferably 250-500 µm (microns), and are present at a concentration of 0.5 to 3% by weight.

Dyes used in the practice of the present invention are generally food color additives presently certified under the Food Drug & Cosmetic Act for use in the food and ingested drugs, including dyes such as FD&C Red No. 3 (sodium salt of tetraiodofluoresecin), FD&C Yellow No.5 (sodium salt of 4-p-sulfophenylazo-l-p-sulfophenyl-5-hydroxypyrazole-3 carboxylic acid), FD&C Yellow No.6 (sodium salt of p-sulfophenylazo-B-naphtol-6-monosulfonate), FD&C Green No. 3 (disodium slat of 4-{[4-(N-ethyl-p-sulffobenzylamino)-phenyl]-(4-hydroxy-2-sulfoniumphenyl)-mewthylene}-[1-(N-ethyl-N-p-sulfobenzyl)-⊗-3,5-cyclohexadienimine], FD&C Blue No. 1 (disodium salt of dibenzyldiethyldiaminotriphenylcarbinol trisulfonic acid of indigotin) and mixtures thereof in various proportions. The concentration of the dye for the most effective result in the present invention is present in the dentifrice composition in an amount from 0.0005 percent to 2 percent of the total weight.

A striped dentifrice product may be obtained using the dual component dentifrice embodiment of the present invention, wherein colorants of contrasting colors are incorporated in each of the dentifrice components to be dispensed; the colorants being pharmacologically and physiologically non-toxic when used in the suggested amounts. Colorants used in the practice of the present invention include both the pigments and dyes discussed above.

Any suitable flavoring or sweetening material may also be incorporated in the dentifrice composition of the present invention. Examples of suitable flavoring constituents are flavoring oils, e.g., oils of spearmint, peppermint, wintergreen, sassafras, clove, sage, eucalyptus, marjoram, cinnamon lemon, and orange, and methyl salicylate. Suitable sweetening agents include sucrose, lactose, maltose, sorbitol, xylitol, sodium cyclamate, perillatine, and sodium saccharin. Suitably, flavor and sweetening agents may together comprise from 0.01% to 5% or more of the preparations.

Antibacterial agents are non-cationic antibacterial agents based on phenolic and bisphenolic compounds, halogenated diphenyl ethers such as Triclosan, benzoate esters and carbanilides as well as cationic antibacterial agents such as chlorhexidine digluconate. Such antibacterial agents can be present in quantities of from 0.03 to 1% by weight of the particular component.

When noncationic antibacterial agents or antibacterial agents are included in any of the dentifrice components, there is also preferably included from 0.05 to 5% of an agent which enhances the delivery and retention of the agents to, and retention thereof on oral surfaces. Such agents useful in the present invention are disclosed in U.S. Patents 5,188,821 and 5,192,531; and include synthetic anionic polymeric polycarboxylates, such as 1:4 to 4:1 copolymers of maleic anhydride or acid with another polymerizable ethylenically unsaturated monomer, preferably methyl vinyl ether/maleic anhydride having a molecular weight (M.W.) of 30,000 to 1,000,000, most preferably 30,000 to 800,000. These copolymers are available for example as Gantrez, e.g. AN 139 (M.W. 500,000), AN 119 (M.W. 250,000) and preferably S-97 Pharmaceutical Grade (M.W. 700,000) available from ISP Technologies, Inc., Bound Brook, N.J. 08805. The enhancing agents when present are present in amounts ranging from 0.05 to 3% by weight.

To prepare the dentifrice components of the present invention, generally the humectants, for example, propylene glycol, polyethylene glycol ingredients, are dispersed with any organic thickeners, sweetener, pigments such as titanium dioxide and any polyphosphates included as anti-calculus ingredients. Water is then added into this dispersion along with any antibacterial agent such as Triclosan, any antibacterial enhancing agent such as Gantrez and any anticalculus additional agents. In the first neutral pH component a fluoride ion source desensitizing agent and phosphate buffeting agent is added. In the second component an ingredient to adjust the pH to an alkaline level is added, such as sodium hydroxide. These ingredients are mixed until a homogenous phase is obtained for each component. Thereafter inorganic thickener, silica abrasive, flavor and surfactant ingredients are added and the ingredients mixed at high speed under vacuum of from 2.66-13.3 kPa (20 to 100 mm of Hg). The resultant product, in the case of each component, is a homogeneous, semi-solid, extrudible paste product.

The dentifrice composition may be applied to hypersensitive tooth surfaces in the form of a paste or gel by tooth brushing or topically applied by being painted directly on the tooth surfaces in the form of a liquid varnish using a soft applicator brush.

The multicomponent dentifrice composition embodiment of the present invention is packaged in a suitable dispensing container in which the components are maintained physically separated and from which the separated components may be dispensed synchronously as a combined ribbon for application to a toothbrush. Such containers are known in the art. An example of such a container is a two compartment dispensing container, such as a pump or a tube, having collapsible sidewalls, as disclosed in U.S. Patents 4,487,757 and 4,687,663; wherein, the tube body is formed from a collapsible plastic web such as polyethylene or polypropylene and is provided with a partition within the container body defiling separate compartments in which the physically separated components are stored and from which they are dispensed through a suitable dispensing outlet

The following example is further illustrative of the present invention, but it is understood that the invention is not limited thereto. All amounts and proportions referred to herein and in the appended claims are by weight, unless otherwise stated.

### Example I

A two component (Component A and B) desensitizing dentifrice of the present invention was prepared, designated "Dentifrice X", Component A having neutral pH (6.5) and a Component B having an alkaline pH (9.5). When combined in equal amounts for tooth brushing, Dentifrice X had a pH of 7.5 in a 1:3 slurry with water. The ingredients of Components A and B are listed in Table I below.

| **TABLE I** | | |
|---|---|---|
| | **Dentifrice X** | |
| | **Weight %** | |
| **Component Ingredients** | **A** | **B** |
| Deionized Water | 32.995 | 36.895 |
| Sodium Fluoride | 1.105* | 1.105* |
| Potassium Nitrate | 5.00 | 5.00 |
| Glycerin | 18.000 | 18.000 |
| Polyethylene glycol 600 | 3.000 | 3.000 |
| Xanthan gum | 7.000 | 7.000 |
| Carboxymethyl cellulose | 0.500 | 0.500 |
| Sorbitol 70% NC | 5.00 | 5.000 |
| Sodium saccharin | 0.400 | 0.400 |
| Titanium Dioxide | --- | 1.000 |
| Pluronic F-127 | 2.000 | 2.000 |
| Sodium Hydroxide (50%) | --- | 1.000 |
| Sodium Phosphate Mono | 4.000 | --- |
| Sodium Phosphate Dibasic | 3.500 | --- |
| FD&C Blue #1 (1.25% solution) | --- | 0.300 |
| Zeodent 115 | 20.000 | 15.000 |
| Zeodent 165 | 1.000 | 1.500 |
| Sodium bicarbonate | --- | 2.500 |
| N-silicate | --- | 3.800 |
| Sodium lauryl sulfate | 1.500 | 1.500 |
| Flavor | 1.100 | 1.100 |
| *Releases 5000 ppm fluoride ion | | |

In the preparation of Dentifrice X, components A and B were prepared wherein the glycerin, polyethylene glycol and organic thickeners were dispersed in a conventional mixer until the mixture became a slurry, which was smooth in appearance. Color and sweetener were dispensed in this slurry before the addition of water. In the preparation of Component A, potassium nitrate was then dispensed in this slurry. In the preparation of Component B, sodium hydroxide was then dispensed in the gel phase. This mixture was mixed for 20 to 30 minutes producing a homogeneous gel phase. The mixture was added to a vacuum mixer and cooled below 40ºC (105°F). Zeodent 115, Zeodent 165 and sodium bicarbonate were then added and mixed for 10 to 30 minutes at high speed under a vacuum of about 6.65 kPa (50 mm Hg), providing a homogenous mixture. The sodium lauryl sulfate and flavor were then added to the individual dentifrice components which was followed by mixing another 5-15 minutes under vacuum of 6.65 kPa (50 mm Hg) to prepare the resultant component product.

The desensitizing efficacy of the two component Dentifrice X was evaluated using 4.25mm X 4.25mm square dentin disks of 750µm thickness cut from extracted human molars. The disks were prepared for treatment by etching with 6% citric acid for 2 minutes to remove any surface smear.

For purposes of comparison the procedure of the Example I was repeated with another group of similarly prepared disks using a dual component dentifrice designated "Dentifrice Y", comparable to that of US 6,180,089 in which the alkaline component designated "Component C" had a pH of 9.5 and the acidic component designated "Component D" had a pH of 5.2. The ingredients of Components C and D of Dentifrice Y are listed in Table II below.

As a control, the procedure of the Example was repeated using a phosphate buffer solution as the treatment which treatment was designated "Control". The ingredients of the phosphate buffer solution are listed in Table III below.

The ingredients of Components C and D of Dentifrice Y are listed in Table II below.

| **TABLE II** | | | |
|---|---|---|---|
| **Dentifrice Y** | | | |
| **Component C** | | **Component D** | |
| **Ingredient** | **%** | **Ingredient** | **%** |
| Deionized water | 29.57 | Dionized water | 25.66 |
| Potassium nitrate | 10.000 | Anhydrous citric acid | 0.531 |
| Glycerin | 25.48 | Sodium citrate | 2.657 |
| PEG 600 | 3.00 | Stannous chloride | 0.600 |
| Xanthan NF | 0.700 | Stannous fluoride | 0.908* |
| Sodium carboxymethyl cellulose | 0.50 | Glycerin | 33.704 |
| Sodium saccharin | 0.4 | Xanthan | 0.500 |
| Titanium dioxide | 2.00 | Sodium carboxymethyl cellulose 2000S | 0.700 |
| Pluoronic F-127 | 1.00 | Sodium saccharin | 0.400 |
| Zeodent 115 | 15.00 | Tetrasodium pyrophosphate | 0.500 |
| Zeodent 165 | 1.75 | FD&C Blue #1 (1.25% soln.) | 0.240 |
| Sodium bicarbonate | 5.00 | PEG 40 oil | 6.00 |
| Sodium hydroxide | 3.00 | Pluoronic F-127 | 2.00 |
| Flavor stannous plus | 1.10 | Zeodent 115 | 20.00 |
| Sodium lauryl sulfate | 1.5 | Zeodent 165 | 3.00 |
| | | Flavor | 1.100 |
| | | Sodium lauryl sulfate | 1.500 |
| | | | |
| **Totals** | **100** | | **100** |

| | | | |
|---|---|---|---|
| *Releases 2200 ppm fluoride ion. Fluoride ion delivery of Dentifrice Y is 1100 ppm when Component C and D are combined for use. | | | |

| **TABLE III** | | |
|---|---|---|
| **Phosphate Buffer Solution** | | |
| **Ingredient** | **Wt. %** | **Millimoles** |
| Sodium phosphate mono | 0.0087 | 0.63 |
| CaCl₂ | 1.1456 | 1.06 |
| NaCl | 0.877 | 150.0 |

The etched disks were then treated by separately brushing the discs for a 60 second period with either Dentifrice X or Y or the phosphate buffer solution (control).

The surface composition of the treated disks were then subjected to Electron Spectroscopy for Chemical Analysis (ESCA) and Scanning Electron Microscopy (SEM) analysis. The ESCA results are recorded in Table IV below as an average for each group. The percentage of nitrogen on the dentin surface is generally attributed to the amount of exposed collagen material which is an integral part of the dentin structure. The reduced amount of nitrogen is indicative of a surface coating, and the higher the amount of calcium ion, the greater the degree of tubular occultation.

| **TABLE IV** | | | | | | | | | | |
|---|---|---|---|---|---|---|---|---|---|---|
| **ESCA Analysis** | | | | | | | | | | |
| **Atomic Percent** | | | | | | | | | | |
| **Dentifrice** | **C** | **O** | **N** | **Ca** | **P** | **Si** | **Na** | **Sn** | **F** | **P/Ca Ration** |
| X | 33.20 | 42.63 | 3.78 | 7.66 | 6.32 | 4.88 | 0.80 | 0.19 | 0.55 | 0.82 |
| Y | 27.76 | 47.27 | 2.45 | 4.73 | 4.10 | 11.39 | 1.02 | 1.16 | 0.11 | 0.86 |
| Control | 59.69 | 22.78 | 14.72 | 1.17 | 0.99 | 0.68 | -- | -- | -- | 0.85 |

The results recorded in Table IV indicate that the amount of deposit formed on the surface of the dentin disks treated with the combined components of Dentifrice X of the present invention is substantially greater than the disks treated with comparative Dentifrice Y, indicating a significantly greater degree of tubular obturation would be experienced with the use of Dentifrice X as compared to Dentifrice Y.

The SEM photomicrographs taken of the dentin surfaces subjected to the brushing treatments are shown in Figures 1-3 respectively. Examination of the SEM of the Dentifrice X treated dentin disk surface, (Figure 1), indicates that dentinal tubule obturation was substantially complete as compared to treatment with comparative dual component Dentifrice Y as indicated by examination of the photomicrograph of Fig. 2. The Control treatment of the dentin disks using an phosphate buffer solution as shown in the SEM of Fig. 3 indicates a limited amount of dentinal tubule obturation.

The ESCA and the SEM results all provide evidence that the unique combination of the neutral and alkaline potassium ion containing dentifrice components in combination with high releasable fluoride ion concentration levels effects an unexpected significant improvement in the remediation of dentinal hypersensitivity.

## Claims

1. A two component dental composition which eliminates or substantially reduces the discomfort and pain associated with dentinal hypersensitivity which composition comprises a first dentifrice component having a neutral pH in the range of 6.5 to 7.5, the pH being buffered with a phosphate salt, a second dentifrice component having an alkaline pH in the range of 8.0 to 9.9, and at least one of the components containing a fluoride and potassium ion releasable salt, the fluoride salt present in the composition delivering a fluoride ion concentration of 1100 to 8800 ppm, the first and second components being maintained separate from each other until dispensed and combined for application to teeth requiring relief from dentine hypersensitivity, whereby heightened desensitization is experienced by the user.

2. The composition of Claim 1 wherein each component contains a fluoride ion and a potassium ion releasable salt.

3. The composition of Claim 1 wherein the potassium ion releasable salt is potassium nitrate.

4. The composition of Claim 1 wherein the fluoride salt present in the dual component composition delivers a fluoride ion concentration of 2500 to 8800 ppm.

5. The composition of Claim 1 wherein the fluoride ion releasable salt is sodium fluoride.

6. The method of Claim 1 wherein the neutral pH dentifrice component contains a sodium phosphate salt buffer.

7. The composition of Claim 1 wherein the pH of the alkaline dentifrice component is adjusted with sodium hydroxide.

8. Use of (1) a first dentifrice component having a neutral pH buffered with a phosphate salt in the range of 6.5 to 7.5 and (2) a second dentifrice component having an alkaline pH in the range of 8.0 to 9.9, at least one of the components containing a desensitizing potassium ion releasable salt and a fluoride ion releasable salt, for eliminating or reducing the discomfort and pain associated with dentinal hypersensitivity, the fluoride salt present in the composition delivering a fluoride ion concentration of 1100 to 8800 ppm, which first and second dentifrice components are in the manufacture of a composition separately housed, dispensing the first and second components and combining the dispensed components for application to teeth requiring relief from dentine hypersensitivity and thereafter applying the combined components to the teeth whereby heightened desensitization is experienced by the user.

9. The use of Claim 8 wherein each component contains a fluoride ion and a potassium ion releasable salt.

10. The use of Claim 8 wherein the potassium ion releasable salt is potassium nitrate.

11. The use of Claim 8 wherein the fluoride salt present in the composition delivers a fluoride ion concentration of 2500 to 8800 ppm.

12. The use of Claim 9 wherein the fluoride ion releasable salt is sodium fluoride.

13. The use of Claim 8 wherein the pH of the alkaline dentifrice component is adjusted with sodium hydroxide.

14. The use of Claim 8 wherein the pH of the neutral pH dentifrice is buffered with a sodium phosphate salt.

## Patentansprüche

1. Zwei-Komponenten-Dentalzusammensetzung, die das mit Dentinüberempfindlichkeit einhergehende Unbehagen und den mit Dentinüberempfindlichkeit einhergehenden Schmerz eliminiert oder wesentlich vermindert, wobei die Zusammensetzung einer erste Zahnpflegemittelkomponente mit einem neutralen pH-Wert im Bereich von 6,5 bis 7,5, wobei der pH-Wert mit Phosphatsalz gepuffert ist, und eine zweite Zahnpflegemittelkomponente mit einem alkalischen pH-Wert im Bereich von 8,0 bis 9,9 umfasst, und mindestens eine der Komponenten Fluorid- und Kaliumionen freisetzendes Salz enthält, wobei das in der Zusammensetzung vorhandene Fluoridsalz eine Fluoridionenkonzentration von 1100 bis 8800 ppm liefert und die erste und die zweite Komponente getrennt voneinander gehalten werden, bis sie zur Anwendung auf Zähnen, die einer Linderung der Dentinüberempfindlichkeit bedürfen, abgegeben und kombiniert werden, wodurch der Anwender erhöhte Desensibilisierung wahrnimmt.

2. Zusammensetzung nach Anspruch 1, wobei jede Komponente Fluoridionen und Kaliumionen freisetzendes Salz enthält.

3. Zusammensetzung nach Anspruch 1, wobei das Kaliumionen freisetzende Salz Kaliumnitrat ist.

4. Zusammensetzung nach Anspruch 1, wobei das in der Zwei-Komponenten-Zusammensetzung vorhandene Fluoridsalz eine Fluoridionenkonzentration von 2500 bis 8800 ppm liefert.

5. Zusammensetzung nach Anspruch 1, wobei das Fluoridionen freisetzende Salz Natriumfluorid ist.

6. Zusammensetzung nach Anspruch 1, wobei die Zahnpflegemittelkomponente mit neutralem pH-Wert Natriumphosphatsalz-Puffer enthält.

7. Zusammensetzung nach Anspruch 1, wobei der pH-Wert der alkalischen Zahnpflegemittelkomponente mit Natriumhydroxid eingestellt ist.

8. Verwendung von (1) einer ersten Zahnpflegemittelkomponente mit einem neutralen pH-Wert, der mit Phosphatsalz auf den Bereich von 6,5 bis 7,5 gepuffert ist, und (2) einer zweiten Zahnpflegemittelkomponente mit einem alkalischen pH-Bereich von 8,0 bis 9,9, wobei mindestens eine der Komponenten ein desensibilisierendes, Kaliumionen freisetzendes Salz und ein Fluoridionen freisetzendes Salz umfasst, um das mit Dentinüberempfindlichkeit einhergehende Unbehagen und den mit Dentinüberempfindlichkeit einhergehenden Schmerz zu eliminieren oder zu vermindern, wobei das in der Zusammensetzung vorhandene Fluoridsalz eine Fluoridionenkonzentration von 1100 bis 8800 ppm liefert, wobei die erste und die zweite Zahnpflegemittelkomponente bei der Herstellung einer Zusammensetzung separat untergebracht sind, die erste und die zweite Komponente abgegeben und die abgegebenen Komponenten zur Anwendung auf Zähnen, die einer Linderung der Dentinüberempfindlichkeit bedürfen, kombiniert werden, und die kombinierten Komponenten danach auf die Zähne aufgebracht werden, wodurch vom Anwender erhöhte Desensibilisierung wahrgenommen wird.

9. Verwendung nach Anspruch 8, wobei jede Komponente Fluoridionen und Kaliumionen freisetzendes Salz enthält.

10. Verwendung nach Anspruch 8, wobei das Kaliumionen freisetzende Salz Kaliumnitrat ist.

11. Verwendung nach Anspruch 8, wobei das in der Zusammensetzung vorhandene Fluoridsalz eine Fluoridionenkonzentration von 2500 bis 8800 ppm liefert.

12. Verwendung nach Anspruch 9, wobei das Fluoridionen freisetzende Salz Natriumfluorid ist.

13. Verwendung nach Anspruch 8, wobei der pH-Wert der alkalischen zahnpflegemittelkomponente mit Natriumhydroxid eingestellt ist.

14. Verwendung nach Anspruch 8, wobei der pH-Wert des Zahnpflegemittels mit neutralem pH-Wert mit Natriumphosphatsalz gepuffert ist.

## Revendications

1. Composition dentaire à deux composants, qui élimine ou réduit nettement l'inconfort et la douleur associés à l'hypersensibilité dentinaire, cette composition comprenant un premier composant de dentifrice ayant un pH neutre dans la plage de 6,5 à 7,5, le pH étant tamponné avec un sel de type phosphate, un deuxième composant de dentifrice ayant un pH alcalin dans la plage de 8,0 à 9,9, et au moins l'un des composants contenant un sel à ions fluorure et potassium libérables, le sel de type fluorure présent dans la composition procurant une concentration en ions fluorure de 1100 à 8800 ppm, les premier et deuxième composants étant maintenus séparés l'un de l'autre jusqu'à ce qu'ils soient distribués et combinés pour une application sur des dents nécessitant la suppression d'une hypersensibilité de la dentine, grâce à quoi une désensibilisation accrue est ressentie par l'utilisateur.

2. La composition de la revendication 1, dans laquelle chaque composant contient un sel à ion fluorure et ion potassium libérables.

3. La composition de la revendication 1, dans laquelle le sel à ion potassium libérable est le nitrate de potassium.

4. La composition de la revendication 1, dans laquelle le sel de type fluorure présent dans la composition à deux composants procure une concentration en ions fluorure de 2500 à 8800 ppm.

5. La composition de la revendication 1, dans laquelle le sel à ion fluorure libérable est le fluorure de sodium.

6. Le procédé de la revendication 1, dans lequel le composant de dentifrice à pH neutre contient un tampon salin de type phosphate de sodium.

7. La composition de la revendication 1, dans laquelle le pH du composant de dentifrice alcalin est ajusté avec de l'hydroxyde de sodium.

8. Utilisation (1) d'un premier composant de dentifrice ayant un pH neutre tamponné avec un sel de type phosphate dans la plage de 6,5 à 7,5, et (2) d'un deuxième composant de dentifrice ayant un pH alcalin dans la plage de 8,0 à 9,9, au moins l'un des composants contenant un sel à ion potassium libérable désensibilisant et un sel à ion fluorure libérable, pour éliminer ou réduira l'inconfort et la douleur associés à l'hypersensibilité dentinaire, le sel de type fluorure présent dans la composition procurant une concentration en ions fluorure de 1100 à 8800 ppm, ces premier et deuxième composants de dentifrice, contenus séparément, entrant dans la production d'une composition, en distribuant les premier et deuxième composants et en combinant les composants distribués pour une application sur des dents nécessitant la suppression d'une hypersensibilité de la dentine, et en appliquant ensuite les composants combinés sur les dents, grâce à quoi une désensibilisation accrue est ressentie par l'utilisateur.

9. L'utilisation de la revendication 8, dans laquelle chaque composant contient un sel à ion fluorure et à ion potassium libérables.

10. L'utilisation de la revendication 8, dans laquelle le sel à ion potassium libérable est le nitrate de potassium.

11. L'utilisation de la revendication 8, dans laquelle le sel de type fluorure présent dans la composition procure une concentration en ions fluorure de 2500 à 8800 ppm.

12. L'utilisation de la revendication 9, dans laquelle le sel à ion fluorure libérable est le fluorure de sodium.

13. L'utilisation de la revendication 8, dans laquelle le pH du composant de dentifrice alcalin est ajusté avec de l'hydroxyde de sodium.

14. L'utilisation de la revendication 8, dans laquelle le pH du dentifrice à pH neutre est tamponné avec un sel de type phosphate de sodium.
